# EUROPEAN PATENT APPLICATION

(11) **EP 1 580 229 A1**
(43) Date of publication of application: **28.09.2005**
(21) Application number: 04290850.9
(22) Date of filing: 22.03.2004
(51) Int. Cl.: C08L 5/00

(54) **Biopolymer compositons and products thereof**

(71) Applicant: Warner-Lambert Company LLC, New Jersey 07950 (US)
(72) Inventor: SCOTT, Robert, B-9100 Sint Niklaas (BE); CADE, Dominique, 68000 Colmar (FR); TARDY, Claire, 68320 Urschenheim (FR); MIRALLES, Marie- Christine, 68420 Eguisheim (FR)
(74) Representative: Tesch, Rudolf

(57) **Abstract**

This invention relates to biopolymer-based compositions and biopolymer-based formed bodies such as film, sheet, capsule, casing or coating-film or spray thereof. In another embodiment, the invention relates to a process for producing a biopolymer-based starting material and biopolymer-based formed bodies such as cited above, for the manufacture of pharmaceutical, veterinary, food or cosmetic products.

## Description

### INTRODUCTION

This invention relates to biopolymer-based compositions and biopolymer-based formed bodies such as film, sheet, capsule, casing or coating-film or spray thereof. In another embodiment, the invention relates to a process for producing a biopolymer-based starting material and biopolymer-based formed bodies such as cited above, for the manufacture of pharmaceutical, veterinary, food, cosmetic products or other products like, more particularly films for wrapping food, aspics or jellies and preferably for pre-dosed formulations like soft or hard capsules, comprising a biopolymer or a blend of biopolymers that are preferably selected from the group of bacterial originated polysaccharide such as pullulan, soligel ® or dextran, and/or plant originated polysaccharide such as starch, starch derivatives, maltodextrin, or cellulose derivatives.

### BACK GROUND OF THE INVENTION

One of the most preferred edible dosage forms for pharmaceutical, dietary supplement and health food products is the two-piece capsules also referred to as hard capsules. Hard capsules are easy to swallow and tasteless shell. Some capsules may be opened up and sprinkled onto food or into water / juice if consumer or patient has difficulty swallowing.
Conventional hard capsules are made with gelatine by dip moulding process. The dip moulding process is based on the setting ability of hot gelatine solutions by cooling. For the industrial manufacture of pharmaceutical capsules, gelatine is most preferred for its gelling, film-forming and surface active properties. The manufacture of hard gelatine capsules by dip moulding process exploits fully its gelling and film-forming abilities. A typical dip moulding process comprises the steps of dipping moulds pins into a hot solution of gelatine, removing the pins from the gelatine solution, allowing the gelatine solution attached on pins to set by cooling drying and stripping the so-formed shells from the pins. The setting of the solution on the mould pins after dipping is the critical step to obtain a uniform thickness of the capsule shell.

This edible dosage form presents some disadvantages. First, the two-piece capsules or hard capsules are used principally for filling solid material such as powders, granules, pellets, tablets or combinations but rarely for filling liquid or suspension of solids into liquids. Then, the range of application of this edible dosage form is reduced to oral administration. Concerning the manufacturing the filling material is filled afterwards by the formulation manufacturers and not by the capsule suppliers.

Therefore, attempts have been made to manufacture alternative delivering system to hard gelatine capsules. One of the most marketed alternatives is the soft gelatine or soft elastic gelatine capsule. These capsules are typically filled with a liquid containing the active ingredient. Because of their soft, elastic character, some patients view these capsules as easier to swallow than conventional tablets or hard gelatine capsules. Since the dosage form is generally swallowed, it is no necessary to flavour or otherwise mask the often unpleasant taste of the pharmaceutical. Soft gelatine capsules are also preferred to bulk liquid because they are easier to transport and they avoid the need for the patient to measure a prescribed amount of the liquid before dosing.

US5916590 describes a film-shaped composition in which therapeutically effective amount of a pharmaceutical is dissolved in a gelling agent such gelatine for producing soft capsule used as pharmaceutical dosage form. The gel may also be used to mould a two-piece hard gelatine capsule.

Soft capsule can be seen as an appropriate alternative to hard capsules. They can be filled with liquids, combination of miscible liquids and suspension of solid into liquids. Also the range of application of this edible dosage form is large. Additionally to oral administration, this dosage form can be used for pessaries, suppositories and as package in cosmetic industry for breath fresheners, perfumes, bath oils and skin cosmetics or in tube form for single dose applications.

Almost every soft capsule is made using the rotary die process patented by Scherer in 1933. Two independent processes take place, often simultaneously, yielding two different materials, the gel mass and the fill material. Both are united in the encapsulation process that produces wet soft capsules. The wet gel mass is manufactured by mixing together and melting, under vacuum, the processing ingredients. At the encapsulation machine, molten gel mass flows through heated transfer tubes and is cast onto chilled drums, forming two separate ribbons. The thickness of the ribbons is carefully controlled and checked periodically throughout manufacture. The gel ribbons traverse through rollers that provide proper alignment of the ribbons and apply lubricant to the both surfaces of the ribbons. Each gel ribbon forms one half of the soft capsule.

Finally soft capsules are formed during the encapsulation step, using the two gel ribbons and the fill material. Lubricated gel ribbons are fed between a pair of counter-rotating dies, the surface of which contains matching pockets of appropriate size and shape that serve as moulds for forming the soft capsules. The die pockets also seal both sides of the soft capsule and cut the formed soft capsule away from the residual gel ribbon. Situated between the ribbons and the rotating dies, the wedge serves three separate functions during the encapsulation process. First, it heats the gel ribbons close to the gel-solution temperature to ensure that melting of the two gel ribbons occurs when the ribbons are pressed together between the dies. Second, the wedge is part of the system that distributes the fill material from a positive displacement pump to each of the pockets. Finally, the wedge, in conjunction with the lubricant, provides a sealing surface against the ribbons to eliminate air and allows a seal to be formed between the shells and fill material without the introduction of air into the product. Then the soft capsules are conveyed to a tumble dryer to initiate drying.

Gelatine is a hydrocolloid that forms a colloidal solution in water, which exhibits a unique combination of useful properties. These properties include water solubility, solution viscosity, thermally-reversible gelation properties and an ability to form strong, clear, and flexible, high-gloss films. Moreover, the gels melt at body temperature and films will dissolve when digested. Gelatine is a protein.

Commercial uses of gelatine have been established in a wide range of industries, including applications in food, pharmaceutical, medical, photographic, cosmetic and technical products. Commercially, one of the major applications for gelatine is in the pharmaceutical industry, in the production of hard and soft capsules, where the ability of gelatine to form clear, flexible, glossy capsule walls is important. The ability of the gelatine capsules to dissolve in the stomach can also be necessary. Gelatine is also used for the micro-encapsulation of oils and vitamins (especially vitamins A and E) for edible and pharmaceutical uses.
Despite the outstanding properties exhibited by gelatine, alternatives to gelatine are currently being sought, particularly in the pharmaceutical industry.
Recently, a global demand by consumer for natural-non-animal origin capsules increases requesting the replacement of gelatine, an animal origin compound. Successful industrial examples are the capsules made of modified cellulose such as hydroxypropyl methylcellulose (HPMC). Another alternatives to gelatine are exo-polysaccharides selected from the group of pullulan, dextran or soligel® and well known for their gel forming and elastic properties.

Pullulan is a natural water-soluble polysaccharide produced by Pullularia pullulans and consisting of repeating units of maltriose linked by α-1,6 bonds for which Hayashibara Inc. had developed mass-production capability in 1973. Pullulan is used for multiple application including foods, pharmaceuticals, cosmetics, primarily due to its membrane-formation characteristics, adhesion, biodegradable capacity and edibility. It is also easy to process into films, sheets and shaped goods and has been referred to as edible plastic. Pullulan hard capsules are easy to swallow, rapidly soluble and can effectively masks taste and odour. Pullulan is pseudo thermoplastic and may thus be compression moulded or extruded at elevated temperature.

JP5065222 describes a soft capsule, capable of stabilizing a readily oxidizable substance enclosed therein, exhibiting easy solubility and being able to withstand a punching production method. The soft capsule is obtained by blending a capsule film substrate such as gelatine, agar or carrageenan with pullulan.

US3784390 corresponding to FR2147112 and GB1374199 discloses that certain mixtures of pullulan with at least one member of the group consisting of amylose, polyvinyl alcohol and/or gelatine can be shaped by compression moulding or extrusion at elevated temperatures or by evaporation of water from its aqueous solutions to form shaped bodies, such as films or coatings. To retain the valuable properties of pullulan to an important extent the mixture should not contain more than 120 percent amylase, 100 percent polyvinyl alcohol and/or 150 percent gelatine based on the weight of the pullulan in the mixture.

The exo-polysaccharides commercialized under the trade name Soligel® imply six neutral sugars one uronic acid in the main chain and one pyruvyl substituent per repeat unit as well as acetyl substituents but not exactly located by Villain-Simonet et al in International Journal of Biological Macromolecules 27 (2000) 65-75. The exo-polysaccharide is produced by a soil bacterial Rhizobium Leguminasorum and is applicable in the cosmetic, food, pharmaceutical and oil industries and may form as indicated in WO9835993 a transparent and elastic gel.

Dextran is a high molecular weight polysaccharides synthesised by micro organisms (i.e:Leuconostoc mesenteroides and Leuconostoc dextranicum) and consist of D glucose linked by 1,6 bonds (and a few _ 1,3 and _ 1,4 bonds). In EP0888778, a capsule formulation comprising gelatine and a polysaccharide such as dextran is prepared by shaping the drug delivery composition into a capsule and filling a biologically active substance into the capsule that may be hard or soft and are prepared by using a pin moulder or a rotary die processes. Disclosed capsules are intended to be used as colon selective drug delivery system.

Although capsules comprising pullulan, dextran and soligel® were mentioned in several patent or applications, it still remains a need for capsule not containing animal-derived ingredients. New capsule ingredients should be capable of forming clear, mechanically strong products, as an alternative to or substitute for gelatine, particularly for edible and ingestible pharmaceutical applications.

Other alternatives to animal-based systems are film forming compositions with plant origin, such as starch or cellulose. Cellulose is chief constituent of all plant tissues and fibbers. It is a carbohydrate, (C6H10O5)n, isomeric with starch, and is convertible into starches and sugars by the action of heat and acids WO01/66082 relates to a gel composition using combination of starch and iota carrageenan for their gelling property.
Maltodextrin is a partially hydrolyzed starch product.

The present invention overcomes this problem by using high molecular weight, water-soluble biopolymer, which are surprisingly capable of producing clear aqueous solutions and products of suitable mechanical strength, and are therefore suitable for use in known methods for the preparation of hard and soft capsules.

The problem to be solved is therefore the provision of an edible film-forming composition containing a biopolymer or a blend of biopolymers, selected from the group of pullulan, soligel®, dextran, maltrodextrin, starch derivatives and cellulose derivatives in replacement to animal gelling components.

### SUMMARY OF THE INVENTION

This invention relates to compositions containing a biopolymer or a blend of biopolymer, selected from bacterial originated polysaccharide or plant originated polysaccharide. More particularly, the invention relates to a film forming composition. The formed films may have variable hardness properties allowing non-limited art of shaping, from soft formed bodies to hard formed bodies with a desired thickness.

Surprisingly, we found that composition comprising:
at least one biopolymer,
at least one plasticizer and
water
has an effective film forming property, allowing the manufacture of starting material and/or formed bodies. Additionally, biopolymer-based composition may also comprise at least one active ingredient that may be a pharmaceutical active ingredient and the like but not limited to.

In another embodiment, the present invention relates to a process for the manufacture of formed bodies, made of the composition of the present invention characterized by the steps of:
a) Processing a mixture containing at least one biopolymer, at least one plasticizer, and water whilst heating and kneading, into a thermo-plastically process able mass in a processing device;
b) Manufacturing of at least one starting material and
c) Re-shaping of the starting material into a formed body in a continuous or intermittent shaping method.

Formed bodies may be films, sheets, capsules, casing or coating-film or spray. Capsules may be hard or soft capsules. Those capsules may be filled with an active ingredient in a solid or liquid form. Hard capsule are obtained by well known dip moulding method and soft capsules by conventional rotary die method, processes briefly described above. The starting material can have variable forms (ball, film, flat and/or square forms)

### DETAILED DESCRIPTION OF THE INVENTION

This invention relates to biopolymer-based compositions containing a biopolymer or a blend of biopolymers. More particularly, the invention relates to a film forming composition. The formed film may have variable hardness properties allowing non-limited art of shaping, from soft formed bodies to hard formed bodies with a desired thickness.

The invention composition is more preferably edible or food acceptable, use for wrapping food or be in contact with food.
The invention composition is more preferably a pharmaceutically acceptable composition.

The composition is used for the manufacture of pharmaceutical, veterinary, food, cosmetic products or other products like

The products made of the invention composition are formed bodies manufactured by the process as described below or using any means and process already known by the skilled person. Those formed bodies can be film, sheet, capsule (soft or hard capsule), casing, coating-film or spray or pre-dosed formulations product and more preferably in a film form. Examples given here are not limited.

A second embodiment, the present invention relates to a process for producing a starting material and formed bodies.

Surprisingly, we found that composition comprising:
at least one biopolymer,
at least one plasticizer and
water
has an effective film forming property, allowing the manufacture of starting material and/or formed bodies that will be detailed below. "Effective film forming property" is defined by the capacity of the composition to remain in a solid form at a specific temperature and preferably at room temperature. Other important property is the disintegration capacity of the film at a specific temperature and preferably at body temperature.. The invention composition is highly processable. As indicated above, the invention provides a composition for the manufacture of formed bodies used as enteric or colonic delivery system. The amount of the composition components will be adjusted in order to obtain specific release location; enteric or colonic.

The biopolymer-based composition does not require use of auxiliary gelling agents and gelling systems such as carrageenans, gellan and pectin and their associate metal ions as promoters of the gel structure. A positive consequence of the absence of the above mentioned gelling agents is that the dissolution of the formed bodies (capsules for example) made from the composition is not affected by presence of ions either in the dissolution media when tested in vitro or in the stomach or intestinal tract when absorbed. It is well documented that when the dissolution medium contains sufficient gel-forming potassium ions, dissociation of the aggregates is delayed. As consequence the gel structure from gellan and carrageenans remains, leading to reduced solubility. When in vitro the buffer medium contains sodium cations, the gel disruption is faster.

A great advantage of the formed bodies of the invention composition such as film over gelatine films for example is the faster preparation time, resulting from the use of the extrusion process versus conventional melting process. With the extrusion process, the film is formed within a few minutes compared to the several hours needed to melt and debbluble a gelatin solution before film casting

Preferably, the biopolymer is bacterial originated polysaccharide or plant originated polysaccharide. Preferred bacterial originated polysaccharide is an external polysaccharide, namely exo-polysaccharide. Suitable exo-polysaccharides include, but are not limited to pullulan, soligel® and dextran. Preferred plant originated polysaccharides include, but are not limited to maltrodextrin, starch and starch derivatives and cellulose derivatives.

According to the present invention, the biopolymers or blend of biopolymers are selected from the group of pullulan, soligel® dextran, maltrodextrin, starch derivatives and cellulose derivatives. The biopolymer may be used in an amount of 30 to 80% of the total weight of the composition. In a preferred embodiment the biopolymer is used in an amount of 40 to 70% of the total weight of the composition. In a more preferred embodiment the biopolymer is used in an amount of 50 to 60% of the total weight of the composition.

According to the present invention, plasticizers or blend of plasticizers are used to make the invention composition more elastic and pliable and are selected from the group consisting of polyalcohol organics acids, hydroxy acids, amines, acid amides, sulphoxides and pyrrolidones. In a preferred embodiment of the present invention the polyalcohol organic acids (or polyhydric alcohols) are selected from the group of sorbitol, mannitol glycerol, xylitol, maltitol, maltisorb, propylene glycol, lactitol, trehalose, sorbitan esters and sorbitol anhydride and blends of.

The plasticizer or a blend of plasticizers can be added in an amount of 0% to 80% or 0% to 40% of the total weight of the composition. In a preferred embodiment, the plasticizer or blend of plasticizers is added in an amount of 5% to 35% or 10% to 30% of the total weight of the composition and in a more preferred embodiment 15% to 25% of the total weight of the composition.

The amount and choice of the plasticizer help to determine the hardness of the final product or in the present case the formed body and may affect the dissolution or disintegration of the invention composition, as well as its physical and chemical stability.

Water is critical to ensure proper processing during gel preparation. The composition of the present invention contains at least 5% of water of the total weight of the composition, in a preferred embodiment 10% to 40% of the total weight of the composition and in a more preferred embodiment 10% to 25% of the total weight of the composition.

The invention composition as described above may additionally contain one opacifier or a blend of opacifiers. The opacifier is added to the invention composition in order to obtain an opaque gel suspension or final product for protecting light sensitive filled or contained active ingredients. The opacifiers may be present in an amount of 0.1 % to 4% of the total weight of the composition and selected from the group of titanium dioxide, calcium carbonate, iron oxide and glycol stearate. A preferred embodiment of the present invention is titanium dioxide.

The composition as described above may additionally contain one pharmaceutically or food acceptable colouring agents or a blend of colouring agents in the range of from 0% to 10 % of the total weight of the invention composition. The colouring agent may be selected from azo-, quinophthalone-, triphenylmethane-, xanthene- or indigo dyes or natural dyes or mixtures thereof. The colouring agents may also be selected from the group of patent blue V, acid brilliant green BS, red 2G, azorubine, ponceau 4R, amaranth, D+C red 33, D+C red 22, D+C red 26, D+C red 28, D+C yellow 10, yellow 2 G, FD+C yellow 5, FD+C yellow 6, FD+C red 3, FD+C red 40, FD+C blue 1, FD+C blue 2, FD+C green 3, brilliant black BN, carbon black, iron oxide black, iron oxide red, iron oxide yellow, riboflavin, carotenes, anthocyanines, turmeric, cochineal extract, chlorophyll, canthaxathin, caramel and betanin or mixture thereof.

The composition is transparent and colourless as the obtained material formed by evaporation of water from an exo-polysaccharides solution and has a high gloss. Formed bodies of exo-polysaccharides are pliable and have folding endurance. The mechanical and optical properties of the material are not impaired by aging and storage at very high or very low relative humidity. It does not become brittle at temperatures as low as ― 10 °C. It does not retain charges of static electricity and does not readily support the growth of micro organisms. Even very thin formed bodies of exo-polysaccharides are almost impermeable to atmospheric oxygen. It has been noticed also that the composition is homogeneous and does not present any unmolten material or particle.

In another embodiment, the present invention relates to a process for the manufacture of formed bodies, from the composition of the present invention characterized by the steps of:
d) Processing a mixture containing at least one biopolymer, at least one plasticizer, and water whilst heating and kneading, into a thermo-plastically process able mass in a processing device;
e) Manufacturing of at least one starting material and
f) Re-shaping of the starting material into a formed body in a continuous or intermittent shaping method.

Formed bodies may be films, sheets, capsules, casing or coating-forming. Capsules may be hard or soft capsules. Those capsules may be filled with an active ingredient in a solid or liquid form. Hard capsule are obtained by well known dip moulding method and soft capsules by conventional rotary die method. Processes are briefly described above.

The process consists in blending at least one biopolymer and at least one plasticizer in water, into a thermo-plastically processable mass in a processing device. The processing temperature shall not exceed 160°C, in a preferred embodiment 140°C and in a more preferred embodiment 100 to 110°C. The processing device has a rotation speed between 150 and 300 rpm. The processing pressure is in a range of 20 to 70 bars. The ready thermo-plastically processable mass is named in the present description starting material. A desired form is given to the thermo-plastically processable mass using adequate means and process already known by the skilled person. The starting material can have variable forms (ball, film, flat, strip and/or square forms) The present invention will be exemplified using as mean an extruder and preferably a twin-screw extruder. In this preferred embodiment the starting material is in a strip-film form. The starting material form and size must be choosing adequately to the next step in the process that is the re-shaping. At this stage of the process the starting material is at such temperature and is such flexible that the desired form can be given to the starting material. The starting material obtained by the present process is at least one extruded strip-film having a thickness of 40 µm to 1000 µm. In a preferred embodiment, the extruded strip-film has a thickness of 250 µm to 850 µm or 400 µm to 750 µm and in a more preferred embodiment, the extruded strip-film has a thickness of 600 µm to 700 µm. Preferably the starting material is in a strip-film form. Resulting from the invention composition and its processing with the extruders is the reduced quantity of water in the obtained films compared to water content above 40 % with other alternative solution composition with modified starches or cellulose derivatives. An obvious benefit of this lower water content of the obtained starting material or preferably a film, is a significantly shorter drying time for the finished products. The residual moisture of the extruded starting material is comprised between 10% and 30% of the total weight of the extruded starting material. Preferably, the residual moisture of the extruded starting material is comprised between of 10% to 25% of the total weight of the extruded starting material

Finally, starting material is re-shaped into a formed body in a continuous or intermittent shaping method. In a preferred embodiment, the two extruded strip-film starting materials are re-shaped into a formed body. Re-shaping can be made by moulding (i.e. dip-moulding of capsules), rotary die (i.e. shaping of soft capsules) or by any means and process already known by the skilled person. Means and process for re-shaping is defined regard the form that is desired to be obtained and regard the use of thereof. In a preferred embodiment, the formed body is selected from the list of film, sheet, capsule, casing and coating. In a more preferred embodiment capsule is a hard or a soft capsule. In another more preferred embodiment the formed body is a film of size such that film can be placed in the oral cavity. The formed body may be used as container for unit dosage form or delivery system. From container for unit dosage form and delivery system is should be understand any products of composition as described in the present invention and containing an active ingredient or substance. Those active ingredients or substances may be food, food additives, pharmaceuticals, chemicals, cosmetics and dye stuffs but also spices, fertilizing combinations, seeds, painting, construction and agricultural products. The active ingredients or substances may be added to the processing mixture of the invention composition or may be filled later on (at the re-shaping stage for example) into the formed body in a solid or liquid form. In case of soft capsule, the filling occurs simultaneously to the shaping process.

Extruded starting material of step b) of the process has an elongation at rupture at room temperature between 100% and 1000%, in a preferred embodiment between 200% and 500% and in a more preferred embodiment between 200% and 300%. Elongation at rupture is considered before drying step.
The Young modulus of extruded starting material is at room condition in the range of 0.5 to 40 MPa, in a preferred embodiment 1 to 10 MPa and in a more preferred embodiment 2 to 4 MPa. The Young modulus is considered before drying step.

The extruded starting material in a flat film form is preferably shaped using the rotary die technology wherein the wedge temperature is comprised between 50 to 100 ° C, preferably 60 to 90°C and more preferred 70 to 85°C.

The processing mixture comprising biopolymer(s), plasticizer(s) and water is as described above.

The processing mixture as described in step a) may additionally contain an opacifier as described above.

The processing mixture as described in step a) may additionally contain a pharmaceutically or food acceptable colouring agents as described above.

In a preferred embodiment relating to capsule, mould-release lubricants are used to facilitate the removal of the mould pins from the capsule-forming core. By "lubricant" is meant a material capable of reducing friction between the mould pins and the inside surface of the formed capsule. The lubricant is compatible with the capsule (i.e., should not degrade the capsule), facilitates removal of the capsule from the mould pins and is pharmaceutically acceptable (i.e., non-toxic). While the lubricant can be a single lubricate compound, it may also be a "lubricant composition" having one or more lubricate compounds and, optionally, other additives or diluents present therein. Many suitable lubricants are available and are used in capsule manufacture. Examples of possible lubricants include: silicone oil; sodium or magnesium lauryl sulfate; fatty acids (e.g., stearic and lauric acid); stearates (e.g., magnesium, aluminium or calcium stearate); boric acid; vegetable oils; mineral oils (e.g. paraffin); phospholipids (e.g., lecithin); polyethylene glycols; sodium benzoate; and mixtures of the above, sodium stearyl fumarate, hydrogenated vegetable oil, hydrogenated castor oil, hydrogenated cottonseed oil, stearic acid and calcium stearate, and the like. Often, other components are present in the lubricant. For example, calcium soap may be dispersed in the oil lubricant. Sometimes, the lubricant is dissolved in petroleum, for example. Such lubricant compositions are well known in the art and are meant to be encompassed by the term "lubricant". The releaser, lubricant or de-moulding agent is used in an amount 0.25 to 1 % of the processing mixture

Finally the formed bodies are dried with any known means and methods. In case of soft capsules this step can be done with air at 30°C in 1 hour. After drying the thickness of the material forming the formed bodies decrease. The thickness is in a range of 250 µm to 500 µm and preferably 300 µm to 400 µm.

As indicated above the formed body is shaped as films, sheets, capsules, casing or coating-film or spray that can be used as container for unit dosage form or delivery system. In a preferred embodiment the formed body may be a coating of tablets, pellets or capsules. In a more preferred embodiment coating is an enteric coating or a colonic coating.

The present invention will now be described with reference to the accompanying examples. These examples are intended to illustrate the invention and the scope of the invention should not be considered to be limited to the embodiment described herein.

### CASE 1:

### Examples of film forming compositions with their extrusion conditions and properties.

### Example 1:

| | |
|---|---|
| Polymer | Pullulan 61.7 % |
| Excipients and manufacturing aids | Sorbitol 23 % |
| | Water 15.3 % |
| Processability | +++ |
| Extrusion temperature °C | 105 |
| Extrusion pressure Bar | 32 |
| Film forming properties | +++ |
| Adhesion to process surfaces | None |
| % Elongation | 360 % |
| Young Modulus | 1 MPa |

### Example 2:

| | |
|---|---|
| Polymer | Pullulan 61.6 % |
| Excipients and manufacturing aids | Sorbitol 15.4 % |
| | Mannitol 3.8 % |
| | Water 19.2 % |
| Processability | +++ |
| Extrusion temperature °C | 105 |
| Extrusion pressure Bar | 38 |
| Film forming properties | +++ |
| Adhesion to process surfaces | None |
| % Elongation | 320 % |
| Young Modulus | 2 Mpa |

### Example 3:

| | |
|---|---|
| Polymer | Pullulan 66.6 % |
| Excipients and manufacturing aids | Sorbitol 15.1 % |
| | Mannitol 3.8 % |
| | Water 14.5 % |
| Processability | +++ |
| Extrusion temperature °C | 105 |
| Extrusion pressure Bar | 35 |
| Film forming properties | +++ |
| Adhesion to process surfaces | None |
| % Elongation | 260 % |
| Young Modulus | 4 MPa |

### Example 4:

| | |
|---|---|
| Polymer | Pullulan 61 % |
| Excipients and manufacturing aids | Sorbitol 17.6 % |
| | Mannitol 4.4 % |
| | Water 17 % |
| Processability | +++ |
| Extrusion temperature °C | 105 |
| Extrusion pressure Bar | 30 |
| Film forming properties | +++ |
| Adhesion to process surfaces | None |
| % Elongation | 300 % |
| Young Modulus | 3.7 MPa |

### Example 5:

| | |
|---|---|
| Polymer | Pullulan 48.4 % |
| | Maltodextrin 12.1 % |
| Excipients and manufacturing aids | Sorbitol 15.8 % |
| | Mannitol 4.0 % |
| | Water 19.7% |
| Processability | +++ |
| Extrusion temperature °C | 95 |
| Extrusion pressure Bar | 26 |
| Film forming properties | +++ |
| Adhesion to process surfaces | None |
| % Elongation | 315 % |
| Young Modulus | 3.2 MPa |

### Example 6:

| | |
|---|---|
| Polymer | Pullulan 60 % |
| | Soligel 3.2 % |
| Excipients and manufacturing aids | Sorbitol 14.7 % |
| | Mannitol 3.7 % |
| | Water 18.4 % |
| Processability | +++ |
| Extrusion temperature °C | 105 |
| Extrusion pressure Bar | 56 |
| Film forming properties | +++ |
| Adhesion to process surfaces | None |
| % Elongation | 240 % |
| Young Modulus | 2.5 MPa |

### Example 7:

| | |
|---|---|
| Polymer | Dextran 64.5 % |
| Excipients and manufacturing aids | Sorbitol 14.2 % |
| | Mannitol 3.6 % |
| | Water 17.8 % |
| Processability | + |
| Extrusion temperature °C | 90 |
| Extrusion pressure Bar | 32 |
| Film forming properties | +++ |
| Adhesion to process surfaces | None |
| % Elongation | 250 % |
| Young Modulus | 2.2 MPa |

### For all Examples:

Pullulan from Hayashibara Japan,
Soligel® from ARD France,
Maltodextrine Glucidex 19 from Roquette France
Dextran 70 from Amersham Bioscience Amersham plc Buckinghamshire United Kingdom
Sorbitol from Neosorb 70/70 from Roquette France,
Mannitol from Roquette France
Processability: (from excellent +++ to poor ---) describes the behaviour of the powder in the powder feeding to the extruder, the overall behaviour during the extrusion process;
Film forming properties ranking is based on the observation of the obtained film (from excellent +++ to poor ---).
% elongation and Young Modulus are measured on the film immediately after the extrusion (no drying step).

### CASE 2:

### Examples of capsules made from film forming compositions with their manufacturing conditions and properties.

The capsules were manufactured under following conditions:

The dry components (powder) of the film composition are feed into the extruder under controlled weight feeding (Loss-in-weight feeder from Brabender Technologie KG, Duisburg Ge); the liquid part of the film composition is added into the extruder with a peristaltic dosing pump (from Watson-Marlow Bredel Inc Wilmington, MA, USA) with accurate control of the flow. The extruder used is a twin screw extruder (from Thermo PRISM, Stone, Staffordshire, England) equipped at its outlet with an extrusion die for strip-film starting material with adjustable slit thickness. The extruded starting material is "pulled" from the extruder by mean of a conveyor belt (from Linatec Feyzin Rhône, France) with adjustable speed.

The starting material is then passing onto the greasing modules of the soft capsule manufacturing machine (from Technophar Windsor, Ontario, Canada), feed onto the die rolls.

As for the conventional soft elastic capsules, the two strip-film starting materials are processed under the wedge block at appropriate temperature for pre heating and pre conditioning of the composition to enable sealing. The capsule are filled and formed following the conventional gelatine soft capsules manufacturing process. The formed capsules are removed from the net and transferred into the tumbler drier with air at 30 °C for 1 hour. Additional drying is performed on trays.

Film thickness was directly measured after capsule forming without drying.

### Example 1:

| | |
|---|---|
| Polymer | Pullulan 61.7 % |
| Excipients and manufacturing aids | Sorbitol 23 % |
| | Water 15.3 % |
| Filled product | Paraffin oil (fluid) from Cooper Melun |
| | France |
| Wedge temperature °C | 70 - 75 |
| Filling pressure Bar | 4 |
| Film thickness before drying | 750 µm |
| Capsule opening time | 2 min 30 sec |
| Dry film thickness | 400 µm |

### Example 2:

| | |
|---|---|
| Polymer | Pullulan 66.6 % |
| Excipients and manufacturing aids | Sorbitol 15.1 % |
| | Mannitol 3.8 % |
| | Water 14.5 % |
| Filled product | Paraffin oil (fluid) from Cooper Melun |
| | France |
| Wedge temperature °C | 60 - 70 |
| Filling pressure Bar | 4 |
| Film thickness before drying | 700 µm |
| Capsule opening time | 2 min |
| Dry film thickness | 350 µm |

### Example 3:

| | |
|---|---|
| Polymer | Pullulan 66.6 % |
| Excipients and manufacturing aids | Sorbitol 15.1 % |
| | Mannitol 3.8 % |
| | Water 14.5 % |
| Filled product | Vitamin E 1000 I.U. from ADM Archer |
| | Daniels Midland Company Decatur, IL, |
| | USA |
| Wedge temperature °C | 90 |
| Filling pressure Bar | 4 |
| Film thickness before drying | 700 µm |
| Capsule opening time | 1 min 30 sec |
| Dry film thickness | 350 µm |

### Example 4:

| | |
|---|---|
| Polymer | Pullulan 61 % |
| Excipients and manufacturing aids | Sorbitol 17.6 % |
| | Mannitol 4.4 % |
| | Water 17 % |
| Filled product | Evening Primerose oil (Mini 9 % GLA) |
| | from Henry Lamotte GmbH Bremen |
| | Germany |
| Wedge temperature °C | 85 - 90 |
| Filling pressure Bar | 4 |
| Film thickness before drying | 650 µm |
| Capsule opening time | 1 min 30 sec |
| Dry film thickness | 320 µm |

### Example 5:

| | |
|---|---|
| Polymer | Pullulan 48.4 % |
| | Maltodextrin 12.1 % |
| Excipients and manufacturing aids | Sorbitol 15.8 % |
| | Mannitol 4.0 % |
| | Water 19.7 % |
| Filled product | Paraffin oil (fluid) from Cooper Melun |
| | France |
| Wedge temperature °C | 65 |
| Filling pressure Bar | 4 |
| Film thickness before drying | 680 µm |
| Capsule opening time | 1 min |
| Dry film thickness | 350 µm |

## Claims

1. Film forming composition comprising at least one biopolymer, at least one plasticizer and water

2. Composition according to claim 1 wherein biopolymer or a blend of biopolymers are bacterial originated polysaccharide

3. Composition according to claim 2 wherein bacterial originated polysaccharide is an exo-polysaccharide and is selected from the group of pullulan, soligel® and dextran

4. Composition according to claim 1 wherein biopolymer or a blend of biopolymers are plant originated polysaccharide.

5. Composition according to claim 4 wherein plant originated polysaccharide is selected from the group of maltrodextrin, starch derivatives or cellulose derivatives.

6. Composition of claims 1 to 5 wherein the composition is preferably a pharmaceutically or food acceptable composition and preferably in a strip-film form

7. Composition according to claim 1 wherein biopolymer is used in an amount of 30 to 80 % of the total weight of the composition, in a preferred amount of 40 to 70 % of the total weight of the composition or in a more preferred amount of 50 to 60 % of the total weight of the composition

8. Composition according to claim 1 wherein plasticizer or blend of plasticizers is selected from the group of polyalcohol organic acids hydroxy acids, amines, acid amides, sulphoxide and pyrrolidones

9. Composition according to claim 8 wherein polyalcohol organic acids are selected from the group of sorbitol, mannitol glycerol, xylitol, maltitol, maltisorb, propylene glycol, lactitol, trehalose, sorbitan esters and sorbitol anhydride or blend of.

10. Composition according to claims 8 and 9 wherein plasticizer is in an amount of 0 to 40 % of the total weight of the composition, in a preferred amount of 5 to 35 % of the total weight of the composition, in an especially preferred amount of 10 to 30 % of the total weight of the composition or in a more preferably amount of 15 to 25 % of the total weight of the composition

11. Composition according to claim 1 wherein water is in amount of at least 5% of the total weight of the composition, in a preferred amount of 10 to 40 % and in a more preferred amount of 10 to 25% of the total weight of the composition

12. Composition according to claim 1, where the composition contains at least one opacifier.

13. Composition according to claim 12 wherein opacifier is selected from the group of titanium dioxide, calcium carbonate, iron oxide or glycol stearate

14. Composition according to claims 12 and 13 wherein opacifier is preferably titanium dioxide

15. Composition according to claims 12 to 14, wherein opacifier or blend of opacifiers are in a range of 0.1 % to 4% of the total weight of the composition

16. Composition according to claim 1, where the composition contains at least one colouring agent.

17. Composition according to claim 16, wherein colouring agent is selected from the group azo-, quinophthalone-, triphenylmethane-, xanthene- or indigo dyes, and natural dyes or mixtures thereof and selected from patent blue V, acid brilliant green BS, red 2G, azorubine, ponceau 4R, amaranth, D+C red 33, D+C red 22, D+C red 26, D+C red 28, D+C yellow 10, yellow 2 G, FD+C yellow 5, FD+C yellow 6, FD+C red 3, FD+C red 40, FD+C blue 1, FD+C blue 2, FD+C green 3, brilliant black BN, carbon black, iron oxide black, iron oxide red, iron oxide yellow, riboflavin, carotenes, anthocyanines, turmeric, cochineal extract, chlorophyll, canthaxathin, caramel or betanin

18. Composition according to claims 16 and 17, wherein colouring agent or mixture of colouring agents are in a range of 0% to 10% of the total weight of the composition

19. Use of the composition according to any preceding claims for manufacturing of starting material and/or formed body

20. Use according to claim 19 wherein said starting material is preferably in a strip-film form

21. Use according to claim 19 wherein said formed body is film, capsule, sheet, casing or coating-film or spray

22. Use according to claim 19 wherein formed body is a container for unit dosage form or a delivery system.

23. Formed body of composition according to claims 1 to 18

24. Formed body and/or starting material of composition according to claims 1 to 18 containing food stuffs, food additives, pharmaceuticals, chemicals, dye stuffs, spices, fertilizing combinations, seeds, cosmetics, painting and construction products and agricultural products

25. Formed body according to claims 23 and 24 wherein said formed body is selected from the list of film, sheet, capsule, casing or coating-film or spray

26. Capsule according to claim 25 wherein said capsule is a hard or a soft capsule.

27. Use of formed body according to claims 23 to 26 as container for unit dosage form or delivery system or enteric or as container for colonic delivery system

28. Process for producing formed bodies of composition according to claims 1 to 18 **characterized by** the steps of:
a) Processing of a mixture containing at least one biopolymer, water at least one plasticizer, and whilst heating and kneading, into a thermo-plastically processable mass in a processing device;
b) Manufacturing at least one starting material, and
c) Re-shaping starting material into a formed body in a continuous or intermittent shaping method.

29. Process according to claim 28 wherein biopolymer or a blend of biopolymers is as defined in claims 2 to 5

30. Process according to claim 28 wherein biopolymer is used in an amount of 30 to 80 % of the total weight of the processing mixture, in a preferred amount of 40 to 70 % of the total weight of the processing mixture or in an especially preferred amount of 50 to 60 % of the total weight of the processing mixture

31. Process according to claim 28 wherein plasticizer or mixture of plasticizers is selected from the group of polyalcohol organic acids hydroxyl acids, amines, acid amines, sulphoxides and pyrrolidsnes

32. Process according to claim 31 wherein polyalcohol organic acids are selected from the group of sorbitol, mannitol, glycerol, xylitol, maltitol, maltisorb, propylene glycol, lactitol, trehalose, sorbitan esters and sorbital anhydride

33. Process according to claim 31 wherein plasticizer is in an amount of 0 to 40 % of the total weight of the processing mixture, in a preferred amount of 5 to 35 % of the total weight of the processing mixture, in an especially preferred amount of 10 to 30 % of the total weight of the processing mixture or in a more preferably amount of 15 to 25 % of the total weight of the processing mixture

34. Process according to claim 28 where the processing mixture a) contains at least 5% of water, preferably 10 to 40%, more preferred 10 to 25% of the total weight of the processing mixture.

35. Process according to claim 28 wherein the composition contains at least one opacifier

36. Process according to claim 35, wherein opacifier is selected from the group of titanium dioxide, iron oxide or glycol stearate

37. Process according to claims 35 to 36, wherein opacifier is preferably titanium dioxide

38. Process according to claim 35, wherein opacifier or mixture of opacifiers are in a range of 0,2% to 0,5% of the total weight of the processing mixture

39. Process according to claim 28, where the composition contains at least one colouring agent

40. Process according to claim 39, wherein colouring agent is selected from the group azo-. Quinophthalone-, triphenylmethane-, xanthene- or indigoid dyes, iron oxides or hydroxides, titanium dioxide or natural dyes or mixtures thereof and also from the following non limited list: patent blue V, acid brilliant green BS, red 2G, azorubine, ponceau 4R, amaranth, D+C red 33, D+C red 22, D+C red 26, D+C red 28, D+C yellow 10, yellow 2 G, FD+C yellow 5, FD+C yellow 6, FD+C red 3, FD+C red 40, FD+C blue 1, FD+C blue 2, FD+C green 3, brilliant black BN, carbon black, iron oxide black, iron oxide red, iron oxide yellow, titanium dioxide, riboflavin, carotenes, anthocyanines, turmeric, cochineal extract, chlorophyll, canthaxathin, caramel or betanin

41. Process according to claims 39 and 40, wherein colouring agent or mixture of colouring agents are in a range of 0% to 10% of the total weight of the processing mixture.

42. Process according to claim 28, where the mixture applied in a), contains lubricant or de-moulding agent.

43. Process according to claim 42, where lubricant or de-moulding agent is selected from silicone oil; sodium or magnesium lauryl sulfate; fatty acids stearates; boric acid; vegetable oils; mineral oils; phospholipids; polyethylene glycols; sodium benzoate; and mixtures of the above, sodium stearyl fumarate.

44. Process according to claims 42 and 43, where the releaser, lubricant or de-moulding agent is used in an amount 0.25 to 1% of the processing mixture

45. Process according to claim 28 where the starting material is an extruded material

46. Process according to claim 28, where the processing device is an extruder and more preferably a twin-screw extruder

47. Process according to claim 28, where the processing temperature does not exceed 160°C, preferred processing temperature is 140°C and especially preferred processing temperature range is 100 to 110°C.

48. Process according to claim 28, where the processing speed is between 150 and 300 rpm

49. Process according to claim 28, where the processing pressure is between 40 to 70 bars

50. Process according to claim 28, where the thickness of starting material in b), is 40 µm to 1000 µm, preferred is 250 µm to 850 µm and especially preferred is 600 µm to 700 µm

51. Process according to claim 28 for the manufacture of starting material and/or formed body

52. Process according to claim 51 wherein said starting material is in a strip-film form

53. Process according to claim 28 wherein said formed body is selected from the group of film, capsule, sheet, casing or coating-film or spray

54. Process according to claim 53 wherein said capsule is a hard or a soft capsule

55. Process according to claim 28 wherein formed body is a container for unit dosage form, a delivery system or enteric or colonic delivery system

56. Process according to claims 28, 51 to 55 wherein formed body and/or starting material of composition according to claims 1 to 15 contain food stuffs, food additives, pharmaceuticals, chemicals, dye stuffs, spices, fertilizing combinations, seeds, cosmetics, painting, construction and agricultural products

57. Process according to claim 28, where the residual moisture of the extruded film in b), is comprised between 10 to 30%, preferably 10 to 25%

58. Process according to claim 28, where the elongation at rupture of the extruded film in b), at room condition, is comprised between 100 to 1000%, preferably 200 to 500%, more preferred 200 to 300%

59. Process according to claim 28, where the Young modulus of the extruded film in b), at room condition, is between 0.5 to 40 MPa, preferably 1 to 10 MPa, more preferred 2 to 4 MPa.

60. Process according to claim 28, where the shaping method in c), is preferably the rotary die technology

61. Process according the claim 28, where the wedge temperature is comprise between 50 to 100°C, preferably 60 to 90°C, more preferred 70 to 85°C.
